# EUROPÄISCHE PATENTSCHRIFT

(11) **EP 0 809 983 B1**
(45) Veröffentlichungstag und Bekanntmachung des Hinweises auf die Patenterteilung: **21.04.2004**
(21) Anmeldenummer: 97102039.1
(22) Anmeldetag: 09.02.1997
(51) Int. Cl.: A61F 2/24

(54) **Mechanische Herzklappe**
Mechanical heart valve
Valve cardiaque mécanique

(30) Priorität: 10.02.1996 DE 19604881
(43) Veröffentlichungstag der Anmeldung: 03.12.1997
(73) Patentinhaber: Cardioberlin GmbH, 10243 Berlin (DE)
(72) Erfinder: Affeld, Klaus, Prof. Dr. Ing, 14050 Berlin (DE)
(74) Vertreter: Linser, Heinz, Physiker

(56) Entgegenhaltungen:
- EP-A- 0 283 413
- DE-A- 2 335 239
- US-A- 4 597 767
- US-A- 4 979 955

## Beschreibung

Die Erfindung betrifft eine mechanische Herzklappe für die Anwendung als künstliches Herz oder als Herzunterstützungssystem oder aber für den Einsatz in künstlichen Konduits, wie sie für eine Verbindung der linken Herzkammer mit der Aorta implantiert werden.

Eine künstliche mechanische Herzklappe hat die Aufgabe, den Blutstrom in der einen Richtung freizugeben und in der umgekehrten Richtung zu verschließen. Dabei soll sie einen möglichst großen freien Querschnitt freigeben, wenn der Druck stromauf größer als stromab ist. In dieser Phase soll der Schließkörper, ein bewegliches Element der mechanischen Herzklappe, der Blutströmung einen möglichst geringen Widerstand bieten. Kehrt sich durch die Wirkung des Herzens dieses Druckverhältnis um, soll die mechanische Herzklappe diesen Querschnitt schnell verschließen, um eine Rückströmung einer größeren Blutmenge zu vermeiden.

Die Werkstoffe, die für den Bau einer solchen mechanischen Herzklappe zur Verfügung stehen, sind den natürlichen Stoffen, aus denen der Körper die blutführenden Elemente aufbaut, weit unterlegen. Die künstlichen Werkstoffe können die Bildung von Blutgerinnseln verursachen, die die Funktion der mechanischen Herzklappe behindern oder auch Blutgefäße weiter stromab verschließen können. Diese Vorgänge werden neben den Werkstoffeigenschaften weiterhin wesentlich auch durch die Blutströmung durch die Herzklappe bestimmt. Besonders nachteilig sind Stagnationsbereiche, in denen das Blut sich nur wenig oder auch zirkulär bewegt. Solche Bereiche sind technisch gesehen Strömungsablösungen und sie entstehen vor allem, wenn sich der Strömungsquerschnitt erweitert oder aber Ausnehmungen, Kanten und Vertiefungen aufweist. Auch in die Strömung hineinragende Elemente wie Schließkörper, Bügel, Achsen oder Stützen sind an ihrer stromab gelegenen Seite mit der Gefahr der Strömungsablösung verbunden.

Es sind zahlreiche mechanische Herzklappen bekannt, bei denen versucht wird, Strömungsablösungen durch strömungsgünstig gestaltete Schließkörper zu vermeiden.

Weiterhin sind mechanische Herzklappen bekannt, bei denen sich der Querschnitt im Bereich ihres Halteringes in Stromrichtung verringert, damit im Bereich der Zapfenlagerung eine beschleunigte Strömung herrscht und so die Gefahr einer Strömungsablösung verringert wird. Jedoch erstreckt sich der Bereich der beschleunigten Strömung nur auf einen kurzen Bereich und schließt nicht den ganzen Bereich der Herzklappe ein.

Allen diesen mechanischen Herzklappen gemeinsam ist, daß der Schließkörper während der Durchströmphase durch Anschläge in seiner Winkelstellung fixiert wird. Gewöhnlich ist der Anstellwinkel des Schließkörpers zur Strömung so groß, daß die Strömung auf der einen Seite des Schließkörpers abreißt und damit eine Strömungsablösung auftritt.

Allen diesen bekannten mechanischen Herzklappen ist weiterhin gemeinsam, daß eine Strömungsablösung an der Hinterkante ihres Halteringes unvermeidlich ist, weil sich an dieser Stelle der Strömungsquerschnitt erweitert. Die Folge ist, daß alle mechanischen Herzklappen, die aus einem nicht optimal blutverträglichen Material gefertigt sind - dies betrifft alle mechanischen Herzklappen - mit einer bestimmten Rate an thromboembolischen Komplikationen verbunden sind, welche dann den Patienten gefährden.

Diese mechanischen Herzklappen sind für den Ersatz der erkrankten natürlichen Herzen entwickelt worden und auf die dabei vorgefundene Geometrie der natürlichen Strömungkanäle wie zum Beispiel die Aortenwurzel abgestimmt. Bei künstlichen Blutpumpen dagegen ist für die Klappen keine anatomisch bestimmte Geometrie vorgegeben und der Strömungskanal um den Schließkörper kann frei gestaltet werden. Das Gleiche gilt für Konduits, die für eine Verbindung der Herzkammer mit der Aorta klinisch eingesetzt werden.

Dokument EP-A-0 283 413 beschreibt eine mechanische Herzklappe mit den Merkmalen des Oberbegriffs von Anspruch 1.

Der Erfindung liegt die Aufgabe zugrunde, die oben erwähnten Nachteile der bisherigen Lösungen zu vermeiden wie beispielsweise die Strömungsablösung und die an sich bekannte Aufgabe auf technisch bessere Weise zu lösen.

Dies wird gemäß der Erfindung dadurch erreicht, daß eine mechanische Herzklappe für künstliche Blutpumpen und Konduits, einen um reale Zapfen oder um eine virtuelle Drehachse drehbaren Schließkörper aufweist, der sich in einem langgestreckten, den gesamten Schließkörper in seiner geöffneten Stellung umschließenden Strömungskanal mit einer S-förmig geformten Mittellinie befindet, wobei der Schließkörper nur durch die Strömung des Blutes betätigbar ist.

Die Strömung im gesamten Bereich der mechanischen Herzklappe wird damit gemäß der Erfindung so geführt, daß der Querschnitt des Strömungskanals sich stetig verkleinert und nicht erweitert, wobei insbesondere auch der Bereich des Schließkörpers mit eingeschlossen ist. Der scheibenförmige Schließkörper kann dabei nach einer der bekannten und bewährten Weisen durch Bügel oder Vorsprünge und Anschläge gelagert sein.

Die mit der Erfindung erzielten Vorteile bestehen insbesondere darin, daß die Strömung so geführt wird, daß Strömungsablösungen im ganzen Bereich der mechanischen Herzklappe vermieden werden.

Die Erfindung wird anhand der Zeichnungen näher erläutert. Hierbei zeigen:
- FIG.1: eine schematische Darstellung der mechanische Herzklappe im Längsschnitt;
- FIG.2: die Darstellung nach Figur 1, ebenfalls im Längsschnitt, wobei im Bereich der Herzklappe die Stromlinien zu Beginn der Rückstromphase dargestellt sind.

Der Schließkörper 1 ist ein flacher Rotationslcörper, der etwa im Drittel seines Durchmessers in einem Zapfen gelagert oder durch eine hier nicht näher beschriebene aber im Prinzip bekannte Konstruktion um eine virtuelle Drehachse 2 drehbar gelagert ist. Der Schließkörper 1 hat einen Anschlag 4, der ihn in geschlossenem Zustand an einer weiteren Bewegung hindert. In der offenen Stellung weist der Schließkörper 1 keinen Anschlag auf und kann sich in seiner Winkelstellung somit frei einstellen und der Strömung vollständig anpassen. Der Schließkörper 1 befindet sich im Strömungskanal 3, der in seinem Querschnitt vom Einstrom 5 zum Ausstrom 6 hin stetig abnimmt. Dadurch wird eine beschleunigte Strömung erzielt, welche die Ausbildung von Ablösungen behindert. Die Anordnung der virtuellen Drehachse senkrecht zur und außerhalb der Symmetrieachse des Schließkörpers 1 bewirkt, daß der Strömungskanal im Bereich des Schließkörpers 1 in einen Bereich 7 mit größerem Widerstand und einen Bereich 8 mit geringerem Widerstand unterteilt wird. An der Hinterkante des Schließkörper 1 vereinigen sich die beiden Ströme. Die unterschiedlichen Geschwindigkeiten führen hier zur Bildung einer Wirbelschicht 9, die sich weiter stromabwärts in kleine Wirbel auflöst. Die freie Beweglichkeit des Schließkörpers 1 verhindert jedoch die Bildung einer Ablösung. Weiterhin besitzt der Strömungskanal 3 eine S-förmig gekrümmte Mittellinie. Diese Form der Krümmung ist für das Schließen der Klappe erforderlich. Ist am Ende der Durchströmphase die Strömung zur Ruhe gekommen und beginnt die Rückströmung, so verhält sich die Strömung wie bei einer reibungsfreien Flüssigkeit. Grenzschichten und Geschwindigkeitsprofile haben sich noch nicht ausgebildet.

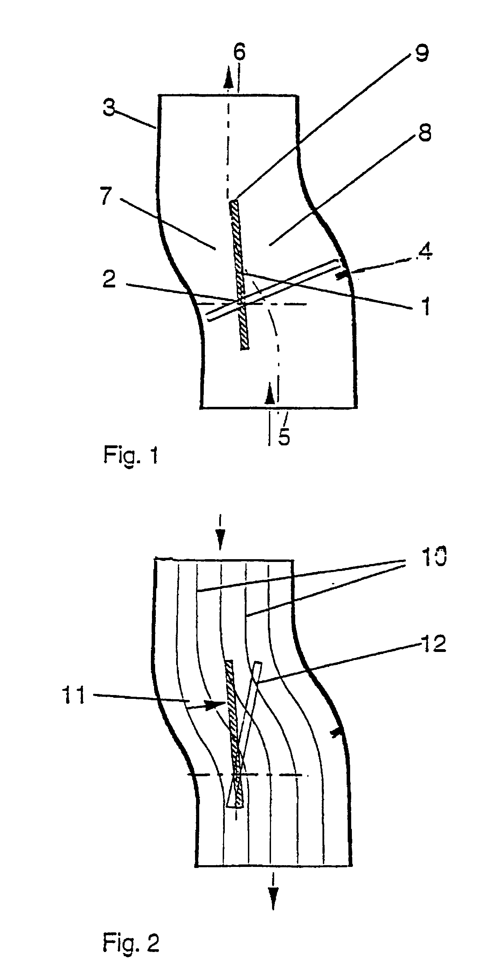

Fig. 2 zeigt die Anordnung der Stromlinien einer solchen Strömung. Die Stromlinien 10 laufen durch die Ebene des Schließkörpers 1 und haben in der Anfangsphase damit eine Komponente 11 senkrecht zur Drehachse des Schließkörpers 1, welche ein Drehmoment auf den Schließkörper ausübt. Damit nimmt die Strömung den Schließkörper 1 mit und dreht ihn etwas weiter in die Strömung in die Position 12, wodurch sich die Mitnahmewirkung weiter verstärkt und die Klappe schnell in die geschlossene Position bis zum Anschlag 4 kommt.

## Patentansprüche

1. Mechanische Herzklappe für künstliche Blutpumpen und Konduits, mit einem um reale Zapfen oder um eine virtuelle Drehachse (2) drehbaren Schließkörper (1), der sich in einem langgestreckten, den gesamten Schließkörper in seiner geöffneten Stellung umschließenden Strömungskanal (3) befindet, wobei der Schließkörper durch die Strömung des Blutes betätigbar ist, **dadurch gekennzeichnet, daß** der Strömungskanal eine S-förmig geformte Mittellinie hat.

2. Mechanische Herzklappe nach Anspruch 1, **dadurch gekennzeichnet, daß** die Ebene, in der die S-förmige Mittellinie liegt, senkrecht zur Drehachse des Schließkörpers ist.

3. Mechanische Herzklappe nach Anspruch 1 oder 2, **dadurch gekennzeichnet, daß** der Schließkörper derartig gelagert ist, daß er während der Durchströmphase sich ohne Anschlagsbegrenzung in die Strömung stellt.

4. Mechanische Herzklappe nach Anspruch 1 oder einem der voranstehenden Ansprüche, **dadurch gekennzeichnet, daß** der Querschnitt des Strömungskanals stromab stetig verkleinert ausgebildet ist.

5. Mechanische Herzklappe nach Anspruch 1 oder einem der voranstehenden Ansprüche, dadurch gekenzeichnet, daß die reale oder virtuelle Drehachse des Schließkörpers sich außerhalb seines Flächenschwerpunlctes befindet.

## Claims

1. Mechanical heart valve for synthetic blood pumps and conduits, with a closing body (1) which can be rotated about real pivots or about a virtual axis of rotation (2) and is in an elongated flow channel (3) which encloses the entire closing body in its opened position, wherein the closing body can be actuated by the flow of the blood, **characterized in that** the flow channel has an S-shaped central line.

2. Mechanical heart valve according to claim 1, **characterized in that** the plane in which the S-shaped central line lies is perpendicular to the axis of rotation of the closing body.

3. Mechanical heart valve according to claim 1 or 2, **characterized in that** the closing body is mounted such that during the flow-through phase it is positioned in the flow without a stop limit.

4. Mechanical heart valve according to claim 1 or one of the preceding claims, **characterized in that** the cross-section of the flow channel is constructed such that it decreases constantly in the downstream direction.

5. Mechanical heart valve according to claim 1 or one of the preceding claims, **characterized in that** the real or virtual axis of rotation of the closing body is outside its planar centre of gravity.

## Revendications

1. Valve cardiaque mécanique pour des pompes à sang et des conduits artificiels, comportant un corps de fermeture (1) pouvant tourner autour de pivots réels ou autour d'un axe de rotation virtuel (2), agencé dans un canal d'écoulement à extension longitudinale, renfermant l'ensemble du corps de fermeture dans sa position ouverte, le corps de fermeture pouvant être actionné par la circulation du sang, **caractérisée en ce que** le canal d'écoulement comporte une ligne médiane en S.

2. Valve cardiaque mécanique selon la revendication 1, **caractérisée en ce que** le plan dans lequel est située la ligne médiane en S est perpendiculaire à l'axe de rotation du corps de fermeture.

3. Valve cardiaque mécanique selon les revendications 1 ou 2, **caractérisée en ce que** le corps de fermeture est agencé de sorte à se placer dans la circulation au cours de la phase d'écoulement, sans limite de butée.

4. Valve cardiaque mécanique selon la revendication 1 ou l'une des revendications précédentes, **caractérisée en ce que** la section transversale du canal d'écoulement est rétrécie en continu vers l'aval.

5. Valve cardiaque mécanique selon la revendication 1 ou l'une des revendications précédentes, **caractérisée en ce que** l'axe de rotation réel ou virtuel du corps de fermeture se trouve hors de son centre de gravité de surface.
